# EUROPÄISCHE PATENTSCHRIFT

(11) **EP 2 018 834 B1**
(45) Veröffentlichungstag und Bekanntmachung des Hinweises auf die Patenterteilung: **29.02.2012**
(21) Anmeldenummer: 08158778.4
(22) Anmeldetag: 23.06.2008
(51) Int. Cl.: A61F 2/86, A61L 31/16

(54) **Degradierbarer Metallstent mit wirkstoffhaltiger Beschichtung**
Degradable metal stent with coating containing active agent
Stent en métal dégradable doté d'un revêtement contenant des additifs

(30) Priorität: 24.07.2007 DE 102007034364
(43) Veröffentlichungstag der Anmeldung: 28.01.2009
(73) Patentinhaber: Biotronik VI Patent AG, 6341 Baar (CH)
(72) Erfinder: Klocke, Björn, 8006, Zürich (CH); Diener, Tobias, 91058, Erlangen (DE); Fringes, Matthias, 91522, Ansbach (DE); Harder, Claus, 91080, Uttenreuth (DE)
(74) Vertreter: Lindner-Vogt, Karin L.

(56) Entgegenhaltungen:
- US-A1- 2004 062 592
- US-A1- 2006 093 643
- US-A1- 2006 198 869
- US-A1- 2007 129 789

## Beschreibung

Die vorliegende Erfindung betrifft einen Stent umfassend oder bestehend aus einem degradierbaren Metallstentgrundkörper und einer wirkstoffhaltigen Schicht, die auf dem Stentgrundkörper aufgebracht ist, ein Verfahren zur Herstellung des erfindungsgemäßen Stents, ein Verfahren zur räumlichen Trennung einerseits von Abbauprodukten eines degradierbaren Metallstentgrundkörpers und andererseits von einem oder mehreren Wirkstoffen, die auf einem erfindungsgemäßen Stent aufgebracht sind, sowie die Verwendung eines erfindungsgemäßen Stents.

Stents im Allgemeinen sind endovaskuläre Prothesen bzw. Implantate, die beispielsweise zur Behandlung von Stenosen verwendet werden. Sie sind außerdem bekannt für die Behandlung von Aneurismen. Stents weisen grundsätzlich eine Tragstruktur auf, die geeignet ist, die Wand eines Gefäßes in geeigneter Weise abzustützen, um so das Gefäß zu weiten bzw. ein Aneurisma zu überbrücken.

Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem superelastischen Metall, wie Nitinol, aufgebaut.

Stents werden derzeit in zwei Grundtypen eingeteilt, die nicht-degradierbaren (dauerhaften) Stents und die (bio)degradierbaren Stents. Nicht-degradierbare Stents sind so ausgestaltet, dass sie im Gefäß für einen unbestimmten Zeitraum verbleiben. (Bio)degradierbare Stents (im Folgenden degradierbare Stents genannt) hingegen werden über einen vorbestimmten Zeitraum hinweg in einem Gefäß abgebaut. Vorzugsweise werden degradierbare Stents erst abgebaut, wenn das traumatisierte Gewebe des Gefäßes verheilt ist, keine Stützfunktion mehr erforderlich ist und somit der Stent nicht weiter im Gefäßlumen verbleiben muss.

Es hat sich allerdings gezeigt, dass durch die Einbringung von Stents in Gefäßsysteme Nebenwirkungen, wie z. B. Restenosen und Thrombosen auftreten können.

Aus diesem Grund wurden Stents entwickelt, die zusätzlich zu Ihrer Tragstruktur ein oder mehrere Wirkstoffe umfassen, die bei oder nach Implantation in das Gefäßsystem an den Organismus abgegeben werden.

Bekannte mit Wirkstoff beschichtete Stents weisen eine Beschichtung auf, wobei die Tragstruktur des Stents üblicherweise sowohl auf der luminalen als auch auf der abluminalen (muralen) Oberfläche mit einem wirkstoffhaltigen Polymer beschichtet werden.

Die Freisetzung der Wirkstoffe aus der Polymersmatrix ergibt sich üblicherweise über Diffusionsprozesse und/oder Erosionsprozesse der Polymermatrix.

Bei Stents, deren luminale und abluminale Oberfläche mit wachstumshemmenden, antiproliferativen Wirkstoff(en) beschichtet vorliegt, hat man festgestellt, dass die Endothelialisierung des Stents (Bewachsen des Stents mit Gefäßzellen) verlangsamt bis verhindert wird und somit das Risiko einer Thrombose erhöht ist.

Bei einem Stent mit Magnesiumlegierung steigt der pH-Wert des in ein Gefäß implantierten Magnesiumstents in unmittelbarer Umgebung der Tragstruktur auf Grund der Mg(OH)₂ Bildung auf Grund der Degradierung an. Eine solche pH-Wert Verschiebung ins Basische kann insbesondere schädlich sein für pH-instabile Wirkstoffe, insbesondere Rapamycin und Paclitaxel.

Bei Verwendung von durch Hydrolyse degradierbarem Polymermaterial (z.B. Polyester) als Matrix für die Wirkstoffbeschichtung sinkt der pH-Wert eines in ein Gefäß implantierten Stents in Folge der Hydrolyse des degradierbaren Polymers und Bildung der entsprechenden Säuren (Milchsäure, Glycolsäure, etc.). Durch die pH-Wert Verschiebung ins Saure kann insbesondere die Zersetzungsgeschwindigkeit eines degradierbaren Metallstentgrundkörpers negativ beeinflusst werden.

Aus US 2006/198869 A1 ist ein Stent bekannt, dessen Stentgrundkörper aus einer degradierbaren Legierung besteht, wobei die Stentgrundkörperoberfläche mit mehreren aus Polymer bestehenden Schichten versehen ist, und wobei eine aus Polymer bestehende Schicht als degradierbare, wirkstoffhaltige Schicht realisiert ist.

Die Aufgabe der vorliegenden Erfindung besteht nun darin, einen verbesserten Wirkstoff beladenen, degradierbaren Metallstent bereitzustellen. Die Verbesserung des Stents soll insbesondere darin bestehen, dass die Stabilität des oder der Wirkstoffe möglichst wenig durch die Abbauprodukte der Stentmaterialien, insbesondere Stentgrundkörpermaterialien, beeinflusst werden und/oder die Endothelialisierung des Stents verbessert wird.

Diese Aufgabe wird durch die erfindungsgemäßen Gegenstände, insbesondere der vorliegenden Patentansprüche gelöst. Bevorzugte Ausgestaltungen werden insbesondere in den abhängigen Ansprüchen dargestellt.

Demzufolge löst ein Stent umfassend oder bestehend aus
a) einem degradierbaren Metallstentgrundkörper,
b) einer Trennschicht, die auf der Oberfläche des Stentgrundkörpers aufgebracht ist, dass zumindest Teile der Oberfläche der luminalen Seite nicht bedeckt sind und
c) einer wirkstoffhaltigen Schicht, die auf die Oberfläche der Trennschicht zumindest teilweise auf der abluminalen Seite des Stentgrundkörpers aufgebracht ist und ein oder mehrere Wirkstoffe und gegebenenfalls ein oder mehrere Polymere umfasst
die erfindungsgemäße Aufgabe, indem die Trennschicht b) über einen Zeitraum, in dem der oder die Wirkstoffe von dem Stent freigesetzt werden und der Stentgrundkörper abgebaut wird, einerseits die Abbauprodukte des Stenzgrundkörpers a) und anderseits den oder die Wirkstoffe sowie die Abbauprodukte der Schicht c) räumlich trennt.

Die Beschichtung des erfindungsgemäßen Stents ist dementsprechend so ausgestaltet, dass die Stentstreben oder Stentstruts nur mit der Trennschicht b) in Kontakt stehen und nicht mit der Wirkstoffschicht c). Dementsprechend ist die Trennschicht b) genau zwischen dem Stentgrundkörper und der Wirkstoffschicht c) angeordnet oder kann in einer weiteren Ausgestaltung über die Trennfläche zwischen Stentgrundkörper und Wirkstoffschicht hinausragen. Bei einem erfindungsgemäßen Stent ist die Trennschicht b) so auf die Oberfläche des Metallstentgrundkörpers aufgebracht, dass zumindest Teile der Oberfläche der luminalen Seite des Stentgrundkörpers nicht mit der Trennschicht b) bedeckt sind, so dass die Degradierung des Stentgrundkörpers an der luminalen Seite bei oder nach Implantation in einen menschlichen oder tierischen Körper einsetzen kann.

Hierbei können die bevorzugten Ausgestaltungen des erfindungsgemäßen Stents alle zusammen, teilweise - in beliebiger Kombination - und einzeln vorhanden sein.

Ein weiterer Gegenstand der vorliegenden Erfindung betrifft ein Verfahren zur Herstellung eines erfindungsgemäßen Stents, umfassend oder bestehend aus folgenden Schritten:
a) Bereitstellung eines degradierbaren Metallstentgrundkörpers und einer ersten Zubereitung, die ein oder mehrere Substanzen umfasst,
b) Beschichtung der Oberfläche des Stentgrundkörpers mit der ersten Zubereitung, sodass eine Trennschicht ausgebildet wird, wobei zumindest Teile der Oberfläche der luminalen Seite nicht bedeckt sind,
c) Bereitstellung einer zweiten Zubereitung, die ein oder mehrere Wirkstoffe und gegebenenfalls ein oder mehrere Polymere und umfasst,
d) Beschichtung der Oberfläche oder von Teilen der Oberfläche, der in Schritt b) gebildeten Trennschicht auf der abluminalen Seite des Stentgrundkörpers mit der zweiten Zubereitung c), sodass eine wirkstoffhaltige Schicht ausgebildet wird.

Es wird des weiteren ein Verfahren zur räumlichen Trennung einerseits von Abbauprodukten eines degradierbaren Metallstentgrundkörpers und andererseits von einem oder mehreren Wirkstoffen, sowie gegebenenfalls von einer oder mehrerer degradierbarer Polymerschichten, die auf einem erfindungsgemäßen Stent aufgebracht sind, während und/oder nach Implantation in einen menschlichen oder tierischem Körper, **dadurch gekennzeichnet, dass** das Verfahren folgende Schritte umfasst oder besteht:
a) Bereitstellung eines erfindungsgemäßen Stents und
b) Implantation des erfindungsgemäßen Stents in einen menschlichen oder tierischen Körper, veröffentlicht.

Darüber hinaus wird die Verwendung eines erfindungsgemäßen Stents mit Trennschicht zur räumlichen Trennung einerseits von Abbauprodukten des Stentgrundkörpers und andererseits von dem oder den Wirkstoffen sowie gegebenenfalls von Abbauprodukten der ersten Polymerschicht während oder nach Implantation des Stents in einen menschlichen oder tierischen Körper, veröffentlicht.

Der vorliegenden Erfindung liegt die Erkenntnis zugrunde, dass durch die erfindungsgemäße Beschichtung des Stents mit einer Trennschicht zwischen Stentgrundkörper und Wirkstoffschicht, gegebenenfalls mit Polymermatrix, eine räumliche Trennung einerseits des oder der Wirkstoffe sowie gegebenenfalls der Abbauprodukte einer Wirkstoff-Polymer-Beschichtung und andererseits der Abbauprodukte des degradierbaren Metallstentgrundkörpers bei Implantation des Stents in ein Gefäß erreicht wird.

Ein Vorteil der vorliegenden Erfindung besteht somit darin, dass bei einem erfindungsgemäßen Stent die Abbauprodukte des Stents keinen Einfluss auf pH-instabile Wirkstoffe ausüben können, da die Trennschicht die Abbauprodukte des Stents von dem oder den Wirkstoffen räumlich trennt. Ein weiterer Vorteil der vorliegenden Erfindung besteht darin, dass, sofern der oder die Wirkstoffe in eine Polymermatrix eingearbeitet sind, die Abbauprodukte der Polymermatrix keinen nachteiligen Effekt auf die Degradationsgeschwindigkeit des degradierbaren Stents haben, da die Trennschicht ebenfalls so ausgestaltet ist, dass die Abbauprodukte einer polymerhaltigen Wirkstoffschicht räumlich von dem Stentgrundkörper getrennt werden.

Schließlich liegt ein weiterer Vorteil der vorliegenden Erfindung darin, dass der erfindungsgemäße Stent schneller endothelialisiert wird, d.h. schneller mit Endotelialzellen (endothelial cells, EC) bewachsen wird, da bekanntermaßen (nicht degradierbare und degradierbare, vorzugsweise magnesiumhaltige) Metalloberflächen im Gegensatz zu mit Rapamycin oder Paclitaxel anti-proliferativ beschichteten Oberflächen in der Regel innerhalb weniger Tage bis Wochen nahezu vollständig mit EC besiedelt werden. Insbesondere hat sich gezeigt, dass Magnesiumoberflächen sogar noch deutlich schneller Endotheliasieren, als Oberflächen aus herkömmlichen metallischen Grundkörpermaterialien, wie medizinischer Stahl (316L) oder Kobalt-Chrom-Legierungen. Durch ein schnelleres und komplettes Überwachsen mit Endothel wird die Gefahr von Spätthrombosen, wie sie insbesondere bei wirkstofffreisetzenden Stents des Standes der Technik beobachtet werden können, verringert.

Ein weiterer Vorteil der schnelleren Endothelialisierung beruht darauf, dass der oder die für die Gefäßwand bestimmte Wirkstoffe nicht oder lediglich in so geringem Maße an das Gefäßlumen abgegeben werden, so dass die Wirkstoffkonzentrationen am gewünschten Ort höher und reproduzierbarer sind.

Die im Nachfolgenden dargestellten bevorzugten Ausgestaltungen des erfindungsgemäßen Stents sind auf die jeweiligen erfindungsgemäßen Gegenstände der vorliegenden Anmeldung anzuwenden.

Gemäß der vorliegenden Erfindung umfassen die Materialien des Stentgrundkörpers üblicherweise degradierbares Metall bzw. degradierbare Metalllegierungen.

### Degradierbarer Metallstent:

Im Sinne der vorliegenden Erfindung bedeutet "degradierbarer Metallstent", dass der Metallstent in physiologischer Umgebung, insbesondere im Gefäßsystem eines menschlichen oder tierischen Körpers degradiert, also so abgebaut wird, dass der Stent seine Integrität verliert.

Vorzugsweise ist der degradierbare metallische Werkstoff eine biokorrodierbare Legierung, wobei die Hauptkomponente der Legierung ausgewählt wird aus der Gruppe Magnesium, Eisen, Zink und Wolfram; insbesondere wird für einen degradierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am Höchsten ist. Ein Anteil der Hauptkomponente beträgt vorzugsweise mehr als 50 Gew.%, weiter bevorzugt mehr als 70 Gew.%. Eine Magnesiumlegierung ist bevorzugt.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch ihrer Abbauprodukte, auszeichnet.

Besonders bevorzugt werden Magnesiumlegierungen der WE-Reihe, insbesondere WE43 sowie Magnesiumlegierungen der Zusammensetzung Seltenerdmetalle 5,5 - 9,9 Gew.%, davon Yttrium 0,0 - 5,5 Gew.% und Rest < 1 Gew.%, der Zirkonium und/oder Silizium enthalten kann, wobei Magnesium den auf 100 Gew.% fehlenden Anteil an der Legierung einnimmt, eingesetzt. Diese Magnesiumlegierungen bestätigten bereits experimentell und in ersten klinischen Versuchen ihre besondere Eignung, d.h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Als Stentgeometrien sind alle möglichen und üblichen Stentgeometrien einsetzbar. Bevorzugt sollten die Steg- bzw. Strutquerschnitte an allen Stellen des Stents gleich sein. Bevorzugt sind die Steg- bzw. Strutquerschnitte im Wesentlichen rechteckig, wodurch die Beschichtung mit der Trennschicht, der wirkstoffhaltigen Schicht und der dritten Polymerschicht, die auch als Topcoat bezeichnet werden kann, vereinfacht wird. Solche Querschnitte sind typisch für Stents, die -üblicherweise - per Laser aus rohrförmigem Ausgangsmaterial zurechtgeschnitten werden. Durch eine anschließende Elektropolitur können jedoch die Kanten abgerundet und die Oberfläche(n) geglättet werden. Alternativ können auch ovale Querschnitte der Stege bzw. Struts des Metallstentgrundkörpers, wie sie bei geschweißten Stents auftreten, die insbesondere durch die Firma Medtronic vertrieben werden, verwendet werden.

Im Sinne der vorliegenden Erfindung bedeutet "luminale Seite der Oberfläche eines Stentgrundkörpers" diejenige Oberfläche eines Stentgrundkörpers, die vorrangig mit dem Gefäßlumen und somit mit der Gefäßflüssigkeit in Kontakt steht. Dies ist, mit anderen Worten, beim typischerweise zylinderförmigen Stent, die Fläche in Richtung der mittigen Zylinderachse.

Im Sinne der vorliegenden Erfindung bedeutet "abluminale Seite des Stentgrundkörpers" diejenige Oberfläche des Stentgrundkörpers, die mit dem Gefäßgewebe in Kontakt steht. Dies ist, mit anderen Worten, beim typischerweise zylinderförmigen Stent, die äußere Zylinderfläche.

Die verschiedenen Ausgestaltungen zur abluminalen Beschichtung des Stentgrundkörpers bzw. bei einer Beschichtung der Oberfläche des Stentgrundkörpers, wobei zumindest Teile der luminalen Oberfläche nicht mit Trennschicht b) (2), wirkstoffhaltiger Schicht c) (3) und dritter Polymerschicht d) (4) beschichtet sind, werden insbesondere in den Figuren dargestellt.

Der oder die Wirkstoffe sollten von dem Stentgrundkörper so an das Gefäßgewebe abgegeben werden, dass sie effektiv gegen Neointimaproliferation wirken können. Beispielsweise sollte Paclitaxel von wenigen Sekunden bis zu einigen Tagen an das Gefäß abgegeben werden. Bei Sirolimus und verwandten Limus-Verbindungen, wie zum Beispiel Zotarolimus, Tacrolimus, Everolimus, Biolimus, insbesondere Biolimus A9, sollte der Wirkstoff über ein paar Wochen an das Gefäß abgegeben werden. Geeignete Elutionskinetiken dieser Wirkstoffe aber auch weiterer erfindungsgemäß einzusetzender Wirkstoffe sind in der Literatur bekannt.

Davon unabhängig sollte die Stentdegradation betrachtet werden, da der Stentgrundkörper seine Funktion in der Abstützung des Gefäßes hat. Ein nennenswerter Kollapsdruck sollte mindestens zwei Wochen, bevorzugt 3 bis 6 Monate aufrecht erhalten werden. Die vollständige Stentdegradation sollte dementsprechend bis zu 24 Monate, vorzugsweise 3 bis 6 Monate, nach Verlust des Kollapsdrucks abgeschlossen sein.

Ein weiterer bevorzugter erfindungsgemäßer Stent umfasst oder besteht zusätzlich aus:
d) einer Polymerschicht, die auf die Oberfläche oder einem Teil der Oberfläche der wirkstoffhaltigen Schicht c) und/oder der Trennschicht b) und/oder des Grundkörpers a) aufgebracht ist.

Diese bevorzugte Ausgestaltung ist vorteilhaft, da zum einen die zusätzliche Polymerschicht dem oder den Wirkstoffen Schutz gegen Abrasion, z.B. während Lagerung oder Implantation, bietet. Hierbei wird diese Polymerschicht d) vorzugsweise formschlüssig abluminal auf die Oberfläche des erfindungsgemäßen Stents aufgetragen.

Bevorzugt ist eine Beschichtung mit der Polymerschicht d) nur auf der abluminalen Seite der Stentoberfläche, da eine zusätzliche luminale Beschichtung mit Polymer die Degradationsgeschwindigkeit des Stentgrundkörpers verzögert oder gegebenenfalls bei Magnesiumlegierungen beschleunigt, da bei hydrolytischer Spaltung geeigneter Polymere der pH-Wert sinkt. Weitere Vorteile der abluminalen Beschichtung nach Schritt d) bestehen in einer Vermeidung eines unerwünschten Anhaftens des Polymers am Trägerballon, (sogenanntes "festkleben") sowie in einer Verringerung des Profils des Stents (Durchmesser des auf den Ballon gecrimpten Stents).

Eine solche Polymerschicht d) (auch Topcoat genannt) bietet des Weiteren dann einen Vorteil, wenn eine bestimmte, vorzugsweise verzögerte Elutionskinetik des oder der Wirkstoffe erforderlich ist. Beispielsweise, wenn trotz abluminaler Elution eine zumindest teilweise mögliche Behinderung der Endothelialisierung durch die antiproliferative Wirkung des Wirkstoffes ausgeschlossen werden soll. Auch die Polymerschicht d), wird vorzugsweise auf der abluminalen Oberfläche, weiter bevorzugt auf der Oberfläche oder Teile der Oberfläche der wirkstoffhaltigen Schicht c) und gegebenenfalls der Trennschicht b) aufgebracht.

Für den Fall, dass Polymere für die wirkstoffhaltige Schicht c) oder die polymerhaltige Schicht d) verwendet werden, werden sie üblicherweise ausgewählt aus der Gruppe bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-co-ethylacrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen; Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-Lactid, Poly D,L Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(1-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

Besonders bevorzugte Polymere für die wirkstoffhaltige Schicht c) oder polymerhaltige Schicht d) der vorliegenden Erfindung sind die oben beschriebenen degradierbaren Polymere, da durch den vollständigen Abbau des oder der Polymere kein körperfremder Bestandteil im Organismus verbleibt.

Ein erfindungsgemäßer Stent ist im Weiteren dann besonders bevorzugt, wenn die Trennschicht b) über den gesamten Zeitraum in dem der oder die Wirkstoffe von dem Stentgrundkörper freigesetzt werden und der Stentgrundkörper abgebaut wird, einerseits die Abbauprodukte des Stentgrundkörpers und andererseits den oder die Wirkstoffe sowie gegebenenfalls die Abbauprodukte der wirkstoffhaltigen Schicht c) und gegebenenfalls der degradablen - insbesondere unter Hydrolyse degradierbaren - Polymerschicht d) räumlich trennt.

Bevorzugte Trennschichtmaterialien für die Trennschicht b) umfassen oder bestehen aus: Siliziumkarbid (SiC), Parylene (Poly-para-Xylylene), insbesondere Parylene N, Parylene C und Parylene D (GALXYL®), Glycocalix, Polysulphon, Silikonkautschuk, Polyurethan, Hydroxylapitit und/oder diamond like carbon.

Bevorzugt liegt die Trennschicht b) als eine dünne Schicht vor, bevorzugt im Bereich von 100nm bis 5 µm, die so ausgestaltet ist, dass im betrachteten Zeitfenster eines erfindungsgemäßen Stents mit nennenswertem Kollapsdruck bzw. einer anhaltenden Wirkstoffelution eine weitgehend inerte Trennschicht darstellt, die einerseits den oder die Wirkstoffe sowie gegebenenfalls die Abbauprodukte der zweiten Schicht c) und gegebenenfalls der dritten Polymerschicht d) räumlich trennt. Darüber hinaus hat die Trennschicht b) bevorzugt gute Hafteigenschaften auf dem Grundkörper sowie mit den Schichten c) und d), ist flexibel und zeichnet sich durch gute Biokompatibilität aus.

Ein erfindungsgemäßer Stent ist weiterhin dann bevorzugt, wenn er sich **dadurch kennzeichnet, dass** der oder die Wirkstoffe geeignet sind zur Prophylaxe und/oder Therapie von In-Stent-Restenose und/oder Gewebeinflammation.

Bevorzugte Wirkstoffe werden insbesondere ausgewählt aus der Gruppe bestehend aus: Lipidregulatoren, Immunsuppressiva, Vasodilatatoren, Calciumkanalblocker, Calcineurininhibitoren, Antiphlogistika, Antünflammatorika, Antiallergika, Oligonucleotide, Estrogene, Endothelbildner, Steroide, Proteine, Peptide, Proliferationshemmer, Analgetika, Antirheumatika und Zytostatika, vorzugsweise Cyclosporin A, Paclitaxel und Limus-Verbindungen, vorzugsweise Sirolimus (Rapamycin), Zotarolimus, Tacrolimus, Biolimus, Everolimus.

Die Beschichtung b) der Oberfläche des Stentgrundkörpers mit der ersten Zubereitung, bevorzugt umfassend SiC, Parylene, Hydroxylapatit und/oder diamond like carbon gemäß des erfindungsgemäßen Verfahrens zur Herstellung des erfindungsgemäßen Stents wird üblicherweise über Plasmaverfahren aufgebracht, wobei die luminale Oberfläche des Stentgrundkörpers so geschützt wird, beispielsweise durch Aufziehen eines Stents auf einen Zylinder, einen Dorn, eine Kanüle etc., dass die luminale Oberfläche des Stentgrundkörpers nicht mit der ersten Zubereitung beschichtet wird. Die Beschichtung mit Parylene wird üblicherweise im Vakuum durch Kondensation aus der Gasphase als bevorzugt porenfreier und transparenter Polymerfilm auf den Stentgrundkörper aufgetragen. Andere bevorzugte Zubereitungen wie Polysulphon, Silikonkautschuk und/oder Polyurethan können durch Sprüh- und Tauchverfahren aufgebracht werden, wobei wiederum die luminale Oberfläche des Stentgrundkörpers geschützt wird.

Die Beschichtung der Oberfläche der Trennschicht aus Schritt b) auf der abluminalen Seite des Stentgrundkörpers mit der zweiten wirkstoffhaltigen Zubereitung c), so dass eine wirkstoffhaltige Schicht c) gemäß dem erfindungsgemäßen Verfahren ausgebildet wird, kann durch übliche Verfahren bewerkstelligt werden. Insbesondere kann hierzu eine reine Wirkstoffschmelze, ein Wirkstoff-Lösungsmittelgemisch oder ein Wirkstoff-Polymergemisch für die üblichen Verfahren, wie z. B. Tauchverfahren (Dippcoating), wobei der Stentgrundkörper auf einen Dorn aufgesteckt ist, Sprühbeschichtung mittels Ein- bzw. Mehrstoffdüse, Rotationszerstäubung und Druckdüsen, Sputtern etc., verwendet werden.

Für eine genaue Dosierung des oder der Wirkstoffe der wirkstoffhaltigen Schicht c) wird die Wirkstoffschmelze, die Wirkstofflösung oder der in ein Polymer inkorporierter Wirkstoff auf den Stentgrundkörper, vorzugsweise mittels üblicher Pipetiertechniken, aufgestrichen. Sofern der Stentgrundkörper Einkerbungen in Form von Dellen oder Körbchen aufweist, wird die wirkstoffhaltige Zubereitung bevorzugt in die Einkerbungen, insbesondere die Dellen oder Körbchen, mittels eines Wirkstoffstrahles dosiert.

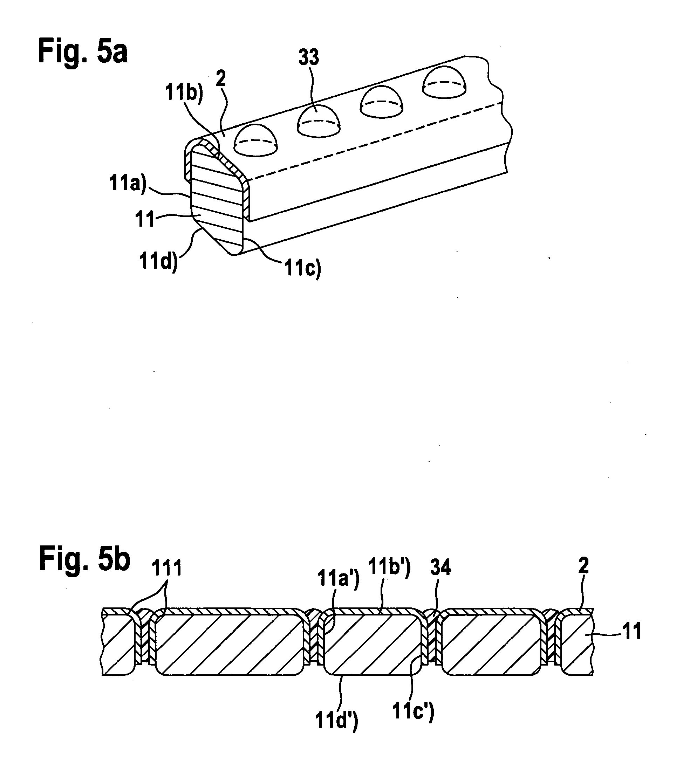

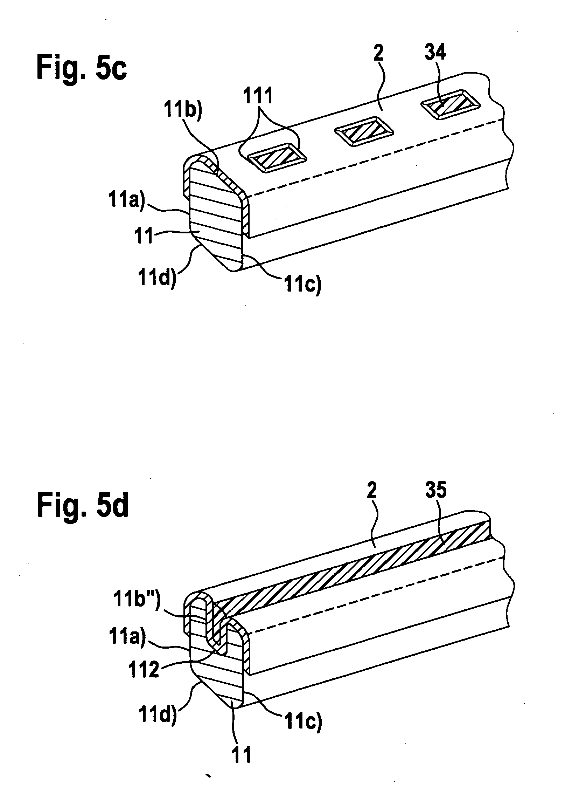

Alternativ kann die wirkstoffhaltige Schicht in Form von (regelmäßig) angebrachten Wirkstoffinseln, vorzugsweise der gleichen Größe auf einer geradlinigen Reihe auf der Trennschicht b) (siehe insbesondere Fig. 5 a) bis d)) auf die Stentstreben bzw. -struts bzw. in geeigneten Vertiefungen insbesondere Löchern gefüllt werden. Die wirkstoffhaltige Schicht c) kann den oder die Wirkstoffe entweder homogen, lagenförmig oder mit einem Konzentrationsgradienten für ein oder mehrere Wirkstoffe gegebenenfalls in einem Wirkstoff-Carrier-Gemisch enthalten.

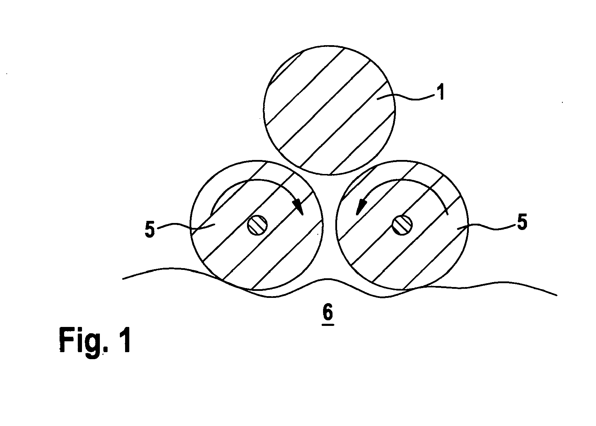

Ein weiteres denkbares Beschichtungsverfahren besteht in einem Walzenauftrag, wie es insbesondere in Fig. 1 dargestellt ist.

Die Beschichtung der Oberfläche des nach einem erfindungsgemäßen Verfahren hergestellten Stents mit der dritten Zubereitung d), die ein oder mehrere Polymere umfasst, so dass eine Polymerschicht d) ausgebildet wird, kann ebenfalls mittels üblicher, bereits oben beschriebener, Verfahren bewerkstelligt werden. Sofern auch hier auf der luminalen Seite kein Polymer vorliegen soll, wird dies **dadurch bewerkstelligt, dass** die luminale Seite der Oberfläche des Stentgrundkörpers so verdeckt wird, dass bei den Beschichtungsverfahren dieser Teil der Oberfläche nicht mit dem Polymer in Kontakt kommt. Bevorzugt wird hierfür ein Zylinder, eine Kanüle oder ein Dorn verwendet, die längs in den Stent eingeführt werden, so dass sie die luminale Seite der Oberfläche des Stentgrundkörpers bedecken und nicht mit der Polymerzubereitung beschichtet wird.

Gegebenenfalls kann einem oder mehreren Beschichtungsschritten ein üblicher Trocknungsschritt oder andere übliche physikalische oder chemische Nachbearbeitungsschritte, z. B. Vakuum- oder Plasmabehandlung, folgen bevor der Stent weiter behandelt wird.

### Figurenbeschreibung:

Die Figuren zeigen zum einen ein erfindungsgemäßes Beschichtungsverfahren gemäß Walzenauftrag und zum anderen Querschnitte der Stentstreben mit erfindungsgemäßen Beschichtungen. Die vorliegende Erfindung wird allerdings nicht auf die hier gezeigten Steg- bzw. Strutgeometrien bzw. auf die gezeigten Anordnungen der Beschichtungen beschränkt.

Von den Figuren ist:
- Fig. 1:: Skizze eines Stents mit Maschinen zum Walzenauftrag und Beschichtungsmedium
- Fig. 2:: Stentstrebe im Querschnitt mit Trennschicht b) und Wirkstoffbeschichtung c)
- Fig. 3:: Stentstrebe im Querschnitt mit Trennschicht b) und Wirkstoffbeschichtung c)
- Fig. 4:: Stentstrebe im Querschnitt mit Trennschicht b) und Wirkstoffbeschichtung c)
- Fig. 5 a):: Perspektivische Sicht auf eine Stentstrebe mit Trennschicht b) und Wirkstoffbeschichtung c)
- Fig. 5 b):: Querschnitt einer Stentstrebe in Längsrichtung mit Trennschichten b) und Wirkstoffbeschichtungen c)
- Fig. 5 c):: Perspektivische Sicht auf eine Stentstrebe mit Trennschicht b) und Wirkstoffbeschichtung c)
- Fig. 5 d):: Perspektivische Sicht auf eine Stentstrebe mit Trennschicht b) und Wirkstoffbeschichtung c)

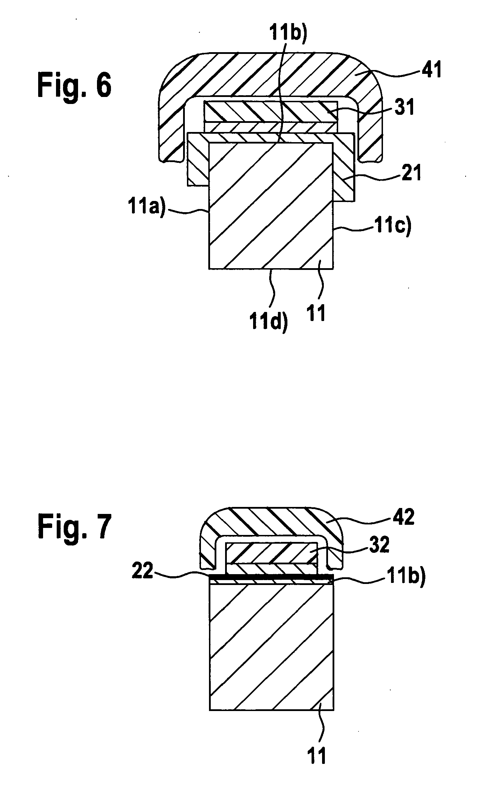

- Fig. 6:: Stentstrebe im Querschnitt mit Trennschicht b), Wirkstoffbeschichtung c) und dritter Polymerbeschichtung d) (Topcoat)
- Fig. 7:: Stentstrebe im Querschnitt mit Trennschicht b), Wirkstoffbeschichtung c) und dritter Polymerbeschichtung d) (Topcoat)

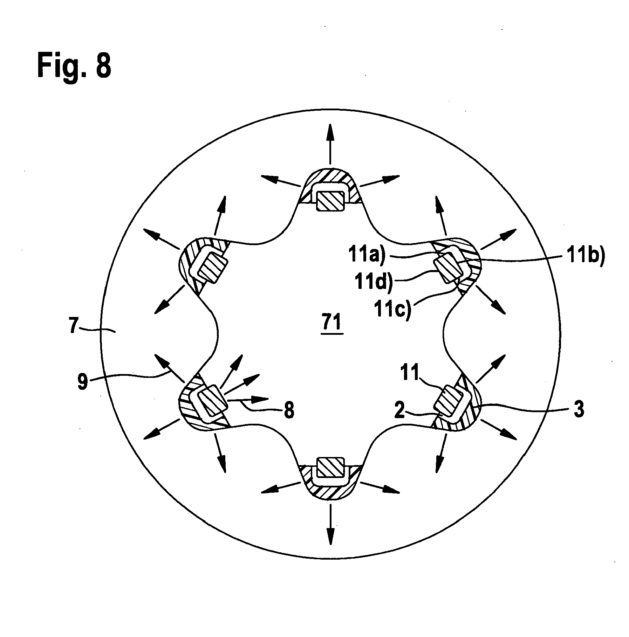

- Fig. 8:: Skizze eines in ein Gefäß implantierten Stents im Querschnitt.

Fig. 1 zeigt einen Stentgrundkörper 1 im Querschnitt sowie zwei Walzen 5 im Querschnitt, wobei die Pfeile die Bewegungsrichtungen der jeweiligen Walzenrollen angeben. Zudem ist das Beschichtungsmedium 6 dargestellt. Das Beschichtungsmedium 6 kann entweder die Zubereitung der wirkstoffhaltigen Schicht oder alternativ die Zubereitung der Polymerschicht des Topcoats darstellen.

Für ein Beschichtungsverfahren gemäß Walzenauftrag wird ein Stentgrundkörper, der bereits eine Trennschicht b) aufweist, mit den beiden gegenläufigen Walzen in Kontakt gebracht. Sobald sich die Walzen in Richtung der Bewegungspfeile drehen, wird das Beschichtungsmedium 6 auf die Oberfläche der Walzen 5 und damit auf die abluminale Oberfläche des Stentgrundkörpers aufgetragen. Vorzugsweise wird der Stentgrundkörper zusätzlich längs zur Achse gedreht, damit die abluminale Oberfläche des Stents vollständig beschichtet werden kann.

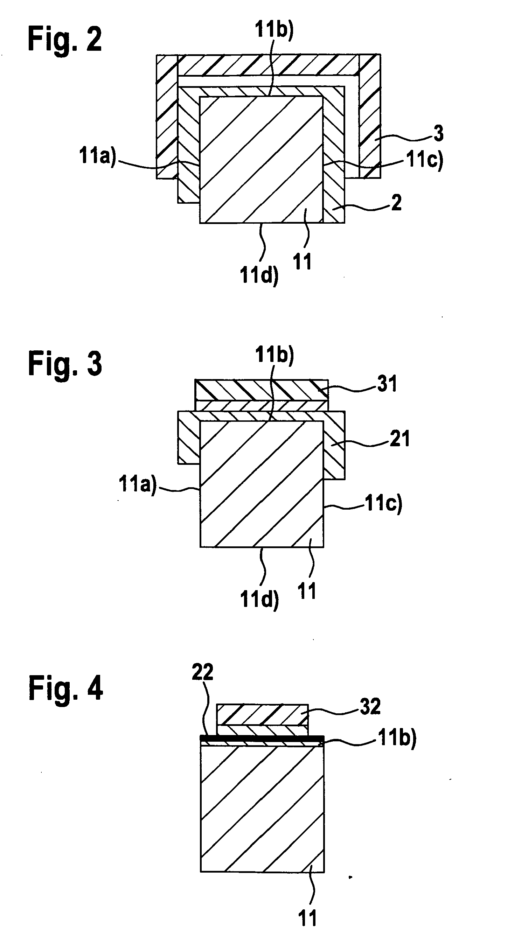

Fig. 2 zeigt eine Stentstrebe 11 eines degradierbaren Metallstents 1, bevorzugt eines Stents aus einer Magnesiumlegierung, im Querschnitt. Die Stentstrebe selbst ist im Wesentlichen rechteckig, wobei die Kanten mittels Elektropolitur, die nach dem Laserzuschnitt durchgeführt wird, abgerundet werden können. Im Weiteren zeigt Fig. 2 eine Trennschicht 2, die an drei Seiten mit der Stentstrebe 11 a), 11 b) und 11 c) in Kontakt steht. Nach Implantation des Stents in ein Gefäß können die Seiten 11 a), 11 b) und 11 c), vorzugsweise 11b) der Stentstrebe mit dem Gefäßgewebe in Kontakt stehen und stellen somit abluminale Oberflächen des Stents dar. Im Gegensatz dazu ist die Seite 11 d) der Stentstrebe dem Gefäßlumen zugewandt, also luminal, und nicht mit der inerten Trennschicht 2 bedeckt. Somit können die Stentstreben eines erfindungsgemäßen Stents von der luminalen Seite 11 d) her degradieren. Die inerte Trennschicht 2 ist vorzugsweise formschlüssig mit der Oberfläche 11 a), 11 b) und 11 c) verbunden.

Darüber hinaus zeigt Fig. 2 die wirkstoffhaltige Schicht 3, die formschlüssig auf die Oberfläche der inerten Trennschicht, die an die Seiten 11 a), 11 b) und 11 c) der Stentstrebe aufgetragen ist, bedeckt. Im dargestellten Beispiel der Fig. 2 bedeckt die wirkstoffhaltige Schicht 3 nicht die gesamte Trennschicht 2, insbesondere nicht in den Endbereichen zur luminalen Seite der Seiten 11 a) und 11 c) der Stentstrebe.

Die wirkstoffhaltige Schicht 3 kann aus ein oder mehreren Wirkstoffen bestehen und gegebenenfalls ein oder mehrere Polymere umfassen.

Fig. 3 zeigt ebenfalls einen Querschnitt einer Stentstrebe 11 eines erfindungsgemäßen Stents 1, wobei die Oberfläche der Seiten 11 a) und 11 b) der Stentstrebe teilweise sowie die gesamte Oberfläche der Stentstrebe 11 b) mit der Trennschicht 21 bedeckt ist. Vorzugsweise ist die Trennschicht 21 formschlüssig mit der Oberfläche der Stentstrebe 11 verbunden. Zudem zeigt Fig. 3 die wirkstoffhaltige Schicht 31, die auf der Oberfläche der Trennschicht 21 im abluminalen Bereich der Seite 11 b) der Stentstrebe aufgebracht ist. Vorzugsweise ist die wirkstoffhaltige Schicht 31 formschlüssig mit der inerten Trennschicht 21 verbunden.

Fig. 4 zeigt ebenfalls einen Querschnitt einer Stentstrebe 11 eines erfindungsgemäßen Stents 1, die eine inerte Trennschicht 22 im Bereich der Oberfläche 11 b) der Stentstrebe aufweist. Zudem zeigt Fig. 4, dass die wirkstoffhaltige Schicht 32 einen Teil der Oberfläche der Trennschicht 22 auf der abluminalen Seite 11 b) der Stentstrebe bedeckt. Auch im vorliegenden Fall bedeckt die wirkstoffhaltige Schicht 32 nicht den gesamten Bereich der inerten Trägerschicht 22.

Fig. 5 a) zeigt eine perspektivische Sicht auf eine Stentstrebe 11 als Teil eines vollständigen Stentdesign 1, wobei die Trennschicht 2, insbesondere auf den Seiten 11 a), 11 b) und 11 c) der Stentstrebe 11 angebracht ist. Zusätzlich weist die Stentstrebe 11 Wirkstoffinseln 23, die auf der Seite 11 b) der Stentstrebe 11 auf der Oberfläche der Trennschicht 2 angebracht sind. Die Wirkstoffinseln 33 liegen in regelmäßig geformten Wirkstoffinseln vor, die in einer geraden Linie auf der Trennschicht 2 angeordnet sind. Eine solche Anordnung erlaubt die Berechnung der Wirkstofffreisetzung nach Implantation. Die Wirkstoffinseln 33 können entweder ein oder mehrere Wirkstoffe gegebenenfalls in einer Trägermatrix homogen verteilt oder in Lagen enthalten und insbesondere kann zu einem oder mehreren Wirkstoffen ein Konzentrationsgradient eingestellt werden.

Fig. 5 b) zeigt einen Querschnitt einer Stentstrebe 11 in Längsrichtung zu einem vollständigen Stentdesign 1, insbesondere Conor^{®}-Stents. Ein solches Stentdesign gemäß Conor^{®}-Stent zeichnet sich dadurch aus, dass die Stentstreben Hohlgänge 111 aufweisen, welche eine Stentstrebe 11 von der abluminalen Seite 11 b') zur luminalen Seite 11 d') durchziehen. Hierbei ist die Trennschicht 2 zum einen an den Seiten 11 a), 11 b) und 11 c) (nicht gezeigt) angebracht und insbesondere an den Seiten 11 a'), 11 b') und 11 c'). Die Wirkstoffbeschichtung 34 liegt in den Hohlgänge 111 der Stentstrebe 11 vor, wobei die Hohlgänge vollständig mit der Wirkstoffbeschichtung 34 oder auch teilweise gefüllt sein können. Die Wirkstoffbeschichtung 34 kann wiederum ein oder mehrere Wirkstoffe gegebenenfalls in einer Trägermatrix in homogener Verteilung oder in Lagen enthalten und insbesondere kann zu einem oder mehreren Wirkstoffen ein Wirkstoffgradient eingestellt werden.

Fig. 5 c) zeigt eine perspektivische Sicht auf eine Stentstrebe 11 eines Stentdesign 1, wie bereits in Fig. 5 b) beschrieben, wobei eine Trennschicht 2 zumindest teilweise auf den Seiten 11 a), 11 b) und 11 d) der Stentstrebe 11 angebracht ist. Die Wirkstoffbeschichtung 34 kann in den Hohlgängen 111 vollständig oder auch teilweise angeordnet sein.

Fig. 5 d) zeigt eine perspektivische Sicht auf eine Stentstrebe 11 als Teil eines vollständigen Stentdesign 1. Die Stentstrebe 11 zeichnet sich dadurch aus, dass sie eine U-förmige Einkerbung 112 aufweist. Die Trennschicht 2 ist zumindest teilweise auf der Oberfläche 11 a), 11 b), 11 b") und 11 c) angebracht. Die Wirkstoffschicht 35 ist in der U-förmigen Einkerbung 112 angeordnet. Die Wirkstoffschicht 35 kann auch nur in einem Teil der U-förmigen Einkerbung vorhanden sein. Auch hier kann die Wirkstoffschichtung 35 ein oder mehrere Wirkstoffe in homogener Verteilung gegebenenfalls in einer Trägermatrix enthalten, aus mehreren Lagen eines oder mehrerer Wirkstoffe bestehen und insbesondere können ein oder mehrere Wirkstoffgradienten eines oder mehrerer Wirkstoffe eingearbeitet werden.

Fig. 6 zeigt eine Stentstrebe gemäß Fig. 3 und zusätzlich eine Polymerschicht 41 als Topcoat, welcher die Oberfläche der wirkstoffhaltigen Schicht 31 sowie teilweise die Oberfläche der inerten Trennschicht 21 an den Seiten 11 a) und 11 b) der Stentstrebe 11 des erfindungsgemäßen Stents 1, vorzugsweise formschlüssig, bedeckt.

Die Polymerschicht 41 des Topcoats kann aus ein oder mehreren Polymeren, vorzugsweise degradierbaren Polymeren bestehen.

Fig. 7 zeigt eine Stentstrebe 11 eines erfindungsgemäßen Stents 1 mit inerter Trennschicht 22 und wirkstoffhaltiger Schicht 32 gemäß Fig. 4 sowie zusätzlich einer Polymerschicht 42 als Topcoat, welcher die Oberfläche der wirkstoffhaltigen Schicht 32 sowie teilweise die Oberfläche der überstehenden inerten Trennschicht 22, vorzugsweise formschlüssig, bedeckt. Die Beschichtung ist nur an der abluminalen Seite 11 b) der Stentstrebe 11 lokalisiert.

In den dargestellten Fig. 2 bis 7 werden erfindungsgemäß beschichtete Stents gezeigt, die so ausgebildet sind, dass der Stentgrundkörper bei bzw. nach Implantation gegebenenfalls verzögert Wirkstoffe an das Gefäßgewebe abgeben kann und gleichzeitig degradieren kann.

Fig. 8 zeigt einen erfindungsgemäßen Stent 1, der in ein Gefäßgewebe 7 implantiert ist, wobei der Stent 1 sechs Stentstreben 11 mit einer luminalen Oberfläche 11 d) und abluminale Oberflächen 11 a), 11 b) und 11 c) aufweist. Die Anzahl der Stentstreben 11 ist variabel. Zusätzlich ist die Stentstrebe 11 auf der abluminalen Oberfläche 11 a), 11 b) und 11 c) mit einer inerter Trennschicht 2 und mit einer wirkstoffhaltigen Schicht 3 beschichtet. Vorzugsweise weist diese Beschichtung eine Polymerschicht als Topcoat 4 gemäß Fig. 6 oder 7 auf.

Mit den Pfeilen 8 ist die Diffusionsrichtung der Degradationsprodukte des erfindungsgemäßen Stentgrundkörpers in das Gefäßlumen 71 dargestellt.

Mit den Pfeilen 9 ist die Diffusionsrichtung des oder der Wirkstoffe der wirkstoffhaltigen Schicht 3 in das Gefäßgewebe 7 dargestellt.

## Patentansprüche

1. Stent umfassend oder bestehend aus:
a) einem degradierbaren Metallstentgrundkörpen (11),
b) einer Trennschicht (2,21,22), die auf der Oberfläche des Stentgrundkörpers so aufgebracht ist, dass zumindest Teile (11d) der Oberfläche der luminalen Seite nicht bedeckt sind und
c) einer wirkstoffhaltigen Schicht (3,31,32,34,35), die auf die Oberfläche der Trennschicht zumindest teilweise auf der abluminalen Seite des Stentgrundkörpers aufgebracht ist und ein oder mehrere Wirkstoffe und gegebenenfalls ein oder mehrere Polymere umfasst,
**dadurch gekennzeichnet, dass** die Trennschicht b) über einen Zeitraum, in dem der oder die Wirkstoffe von dem Stent freigesetzt werden und der Stentgrundkörper abgebaut wird, einerseits die Abbauprodukte des Stentgrundkörpers und andererseits den oder die Wirkstoffe sowie die Abbauprodukte der Schicht c) räumlich tren nt.

2. Stent gemäß Anspruch 1, **dadurch gekennzeichnet, dass** die Trennschicht b) nicht auf der Oberfläche (11d) der luminalen Seite des Stentgrundkörpers aufgebracht ist.

3. Stent gemäß einem der vorstehenden Ansprüche zusätzlich umfassend oder bestehend aus:
d) einer Polymerschicht (4,41,42), die auf die Oberfläche oder einem Teil der Oberfläche der wirkstoffhaltigen Schicht c) und/oder der Trennschicht b) und/oder des Stentgrundkörpers aufgebracht ist.

4. Stent gemäß einem der vorstehenden Ansprüche, **dadurch gekennzeichnet, dass** das oder die Polymere der Schicht c) und/oder der Polymerschicht d) ausgewählt werden aus der Gruppe bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-co-ethyl-acrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen, Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-Lactid, Poly D,L Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(I-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

5. Stent gemäß einem der vorstehenden Ansprüche, **dadurch gekennzeichnet, dass** das degradierbare Metall oder die degradierbare Metalllegierung eine biokorrodierbare Legierung darstellt, die Magnesium, Eisen, Zink und/oder Wolfram umfasst.

6. Stent gemäß Anspruch 5, **dadurch gekennzeichnet, dass** die Legierung eine Magnesiumlegierung ist.

7. Stent gemäß Anspruch 5 oder 6, **dadurch gekennzeichnet, dass** die Magnesiumlegierung Yttrium umfasst.

8. Stent gemäß einem der vorstehenden Ansprüche, **dadurch gekennzeichnet, dass** die Trennschicht b) Siliziumkarbid (SiC), Parylene (Poly-para-Xylylene), Glycocalix, Polysulphon, Silikonkautschuk, Polyurethan, Hydroxylapitit und/oder diamond like carbon umfasst oder daraus besteht.

9. Stent gemäß einem der vorstehenden Ansprüche, **dadurch gekennzeichnet, dass** die Trennschicht b) eine Dicke von 100 nm bis 5 µm aufweist.

10. Stent gemäß einem der vorstehenden Ansprüche, **dadurch gekennzeichnet, dass** der oder die Wirkstoffe geeignet sind zur Prophylaxe und/oder Therapie von In-Stent-Restenose und/oder Gewebeinflammation und/oder ausgewählt werden aus der Gruppe bestehend aus:
Lipidregulatoren, Immunsuppressiva, Vasodilatatoren, Calciumkanalblocker, Calcineurininhibitoren, Antiphlogistika, Antiinflammatorika, Antiallergika, Oligonucleotide, Estrogene, Endothelbildner, Steroide, Proteine, Peptide, Proliferationshemmer, Analgetika, Antirheumatika und Zytostatika.

11. Verfahren zur Herstellung eines Stents gemäß einem der vorstehenden Ansprüche, umfassend oder bestehend aus folgenden Schritten:
a) Bereitstellung eines degradierbaren Metallstentgrundkörpers und einer ersten Zubereitung, die ein oder mehrere Substanzen umfasst,
b) Beschichtung der Oberfläche des Stentgrundkörpers mit der ersten Zubereitung, so dass eine Trennschicht ausgebildet wird, wobei zumindest Teile der Oberfläche der luminalen Seite nicht bedeckt wird,
c) Bereitstellung einer zweiten Zubereitung, die ein oder mehrere Wirkstoffe und gegebenenfalls ein oder mehrere Polymere umfasst und
d) Beschichtung der Oberfläche oder Teile der Oberfläche der Trennschicht aus Schritt b) auf der abluminalen Seite des Stentgrundkörpers mit der zweiten Zubereitung c), sodass eine wirkstoffhaltige Schicht ausgebildet wird.

12. Verfahren gemäß Anspruch 11, **dadurch gekennzeichnet, dass** in Verfahrensschritt b) die Oberfläche des Stentgrundkörpers auf der abluminalen Seite mit der ersten Zubereitung beschichtet wird, sodass auf der abluminalen Seite des Stentgrundkörpers die Trennschicht b) ausgebildet wird und die luminale Seite nicht mit der Trennschicht b) beschichtet ist.

13. Verfahren gemäß Anspruch 11 oder 12, **dadurch gekennzeichnet, dass** das Verfahren zusätzlich folgende Schritte umfasst:
e) Bereitstellung einer dritten Zubereitung umfassend ein oder mehrere Polymere und
f) Beschichtung der Oberfläche des gemäß Anspruch 11 oder 12 hergestellten Stents mit der Zubereitung, sodass eine dritte Polymerschicht ausgebildet wird, die die Oberfläche oder Teile der Oberfläche der wirkstoffhaltigen Schicht d) und/oder der Trennschicht b) und/oder der Stentgrundkörper ohne Beschichtung bedeckt.

## Claims

1. A stent comprising or consisting of:
a) a degradable metal stent base body (11),
b) a separating layer (2, 21, 22), which is applied to the surface of the stent base body so that at least parts (11d) of the surface of the luminal side are not covered; and
c) an active agent-containing layer (3, 31, 32, 34, 35), which is applied at least to a part of the surface of the separating layer on the abluminal side of the stent base body and comprises one or more active agents and optionally one or more polymers,
**characterized in that** the separating layer b) spatially separates the decomposition products of the stent base body on the one hand, and the active agent or agents and the decomposition products of the layer c) on the other hand, over a period during which the active agent is, or active agents are, released by the stent and the stent base body is decomposed.

2. The stent according to claim 1, **characterized in that** the separating layer b) is not applied to the surface (11d) of the luminal side of the stent base body.

3. A stent according to any one of the preceding claims, additionally comprising or consisting of:
d) a polymer layer (4, 41, 42), which is applied to the surface, or a part of the surface, of the active agent-containing layer c) and/or of the separating layer b) and/or of the stent base body a).

4. A stent according to any one of the preceding claims, **characterized in that** the polymer or polymers of the layer c) and/or of the polymer layer d) is or are selected from the group consisting of:
- non-degradable polymers: polyethylene; polyvinyl chloride; polyacrylate; preferably polyethyl and polymethyl acrylates, polymethyl metacrylate, polymethyl-co-ethyl acrylate and ethylene/ethyl acrylate; polytetrafluoroethylene, preferably ethylene/chlorotrifluoroethylene copolymers, ethylene/tetrafluoroethylene copolymers; polyamide, preferably polyamide-imide, PA-11, PA-12, PA-46, PA-66; polyetherimide; polyethersulfone; poly(iso)butylene; polyvinyl chloride; polyvinyl fluoride; polyvinyl alcohol; polyurethane; polybutylene terephthalate; silicones; polyphosphazene; polymer foams, preferably polymer foams made of carbonates and styrenes; copolymers and/or blends of the listed polymer classes, polymers of the class of thermoplastics; and
- degradable polymers: polydioxanone; polyglycolide; polycaprolactone; polylactides, preferably poly-L-lactide, poly-D,L-lactide, and copolymers as well as blends thereof, preferably poly(L-lactide-co-glycolide), poly(D,L-lactide-co-glycolide), poly(L-lactide-co-D,L-lactide), poly(L-lactide-co-trimethylene carbonate); triblock copolymers; polysaccharides, preferably chitosan, levan, hyaluronic acid, heparin, dextran, cellulose; polyhydroxy valerate; ethyl vinyl acetate; polyethylene oxide; polyphosphoryl choline; fibrin; albumin; polyhydroxybutyric acid, preferably atactic, isotactic and/or syndiotactic polyhydroxybutyric acid and the blends thereof.

5. A stent according to any one of the preceding claims, **characterized in that** the degradable metal, or the degradable metal alloy, constitutes a biocorrodible alloy comprising magnesium, iron, zinc and/or tungsten.

6. The stent according to claim 5, **characterized in that** the alloy is a magnesium alloy.

7. The stent according to claim 5 or 6, **characterized in that** the magnesium alloy comprises yttrium.

8. A stent according to any one of the preceding claims, **characterized in that** the separating layer b) comprises or consists of silicon carbide (SiC), parylene (poly-para-xylylene), glycocalix, polysulfone, silicon rubber, polyurethane, hydroxyl apatite and/or diamond-like carbon

9. A stent according to any one of the preceding claims, **characterized in that** the separating layer b) has a thickness of 100 nm to 5 µm.

10. A stent according to any one of the preceding claims, **characterized in that** the active agent is, or the active agents are, suitable for the prophylaxis and/or treatment of in-stent restenosis and/or tissue inflammation and/or are selected from the group consisting of:
lipid regulators, immunosuppressants, vasodilators, calcium channel blockers,
calcineurin inhibitors, antiphlogistics, anti-inflammatory agents, anti-allergic drugs, oligonucleotides, estrogens, endothelial forming agents, steroids, proteins, peptides,
proliferation inhibitors, analgesics, antirheumatism agents and cytostatic drugs.

11. A method for producing a stent according to any one of the preceding claims, comprising or consisting of the following steps:
a) providing a degradable metal stent base body and a first formulation comprising one or more substances;
b) coating the surface of the stent base body with the first formulation so that a separating layer is formed, wherein at least parts of the surface of the luminal side are not covered;
c) providing a second formulation comprising one or more active agents and optionally one or more polymers; and
d) coating the surface or parts of the surface of the separating layer from step b) on the abluminal side of the stent base body with the second formulation c), whereby a layer containing an active agent is formed.

12. The method according to claim 11, **characterized in that** in method step b) the surface of the stent base body on the abluminal side is coated with the first formulation, so that the separating layer b) is formed on the abluminal side of the stent base body and the luminal side is not coated with the separating layer b).

13. The method according to claim 11 or 12, **characterized in that** the method additionally comprises the following steps:
e) providing a third formulation comprising one or more polymers; and
f) coating the surface of the stent produced according to claim 11 or 12 with the formulation, so that a third polymer layer is formed which covers the surface, or parts of the surface, of the active agent-containing layer d) and/or of the separating layer b) and/or the stent base body without coating.

## Revendications

1. Stent comprenant ou étant constitué de :
a) un corps de base de stent métallique (11),
b) une couche de séparation (2, 21, 22) appliquée de telle manière sur la surface du corps de base de stent, qu'au moins des parties (11d) de la surface de la face luminale ne sont pas couvertes, et
c) une couche contenant des substances actives (3, 31, 32, 34, 35) appliquée sur la surface de la couche de séparation, au moins partiellement sur la face abluminale du corps de base de stent, et comprenant une ou plusieurs substances actives et le cas échéant un ou plusieurs polymères,
**caractérisé en ce que** sur une période de temps où la ou les substances actives sont libérées par le stent et où le corps de base de stent se décompose, la couche de séparation b) sépare spatialement d'une part les produits de décomposition du corps de base de stent et d'autre part la ou les substances actives ainsi que les produits de décomposition de la couche c).

2. Stent selon la revendication 1, **caractérisé en ce que** la couche de séparation b) n'est pas appliquée sur la surface (11d) de la face luminale du corps de base de stent.

3. Stent selon l'une des revendications précédentes, comprenant ou étant en outre constituée de :
d) une couche polymère (4, 41, 42) appliquée sur la surface ou sur une partie de la surface de la couche contenant des substances actives c) et/ou de la couche de séparation b) et/ou du corps de base de stent.

4. Stent selon l'une des revendications précédentes, **caractérisé en ce que** le ou les polymères de la couche c) et/ou de la couche polymère d) est/sont sélectionné(s) à partir du groupe constitué de :
- polymères non dégradables : le polyéthylène ; le chlorure de polyvinyle ; les polyacrylates ; de préférence les polyacrylates d'éthyle et de méthyle, le polyméthacrylate de méthyle, le polyacrylate de méthyle-co-éthyle et l'éthylène/acrylate d'éthyle ; le polytétrafluoréthylène, de préférence les copolymères d'éthylène/chlortrifluoréthylène, les copolymères d'éthylène/tétra-fluor-éthylène ; le polyamide, de préférence le polyamide-imide, le PA-11, le PA-12, le PA-46, le PA-66 ; le polyétherimide ; le polyéther sulfone ; le poly-iso-butylène ; le polychlorure de vinyle ; le poly-fluorure de vinyle ; l'alcool polyvinylique ; le polyuréthane ; le poly-butylène téréphtalate ; le silicone ; le polyphosphazène ; les mousses de polymères, de préférence les mousses de polymères en carbonates, styrènes ; et/ou des combinaisons des classes de polymères citées, des polymères de la classe des thermoplastes, et les
- polymères dégradables : le polydioxanon ; le polyglycolide ; le polycaprolactone ; les polylactides, de préférence le poly-L-lactide, le poly D,L lactide, et des copolymères ainsi que des combinaisons de ceux-ci, de préférence le poly(L-lactide-co-glycolide), le poly(D,L-lactide-co-glycolide), le poly(L-lactide-co-D,L-lactide), le poly(I-lactide-co-triméthylène carbonate) ; les copolymères triblocs ; les polysaccarides, de préférence le chitosan, le levan, l'acide hyaluronique, l'héparine, le dextran, la cellulose ; le polyhydroxyvalérate ; l'éthyle-acétate de vinyle ; l'oxyde de polyéthylène ; la polyphosphorylcholine ; la fibrine ; l'albumine ; les acides polyhydroxy-butyriques, de préférence les acides polyhydroxy-butyriques atactiques, isotactiques et/ou syndiotactiques, ainsi que des combinaisons de ceux-ci.

5. Stent selon l'une des revendications précédentes, **caractérisé en ce que** le métal dégradable ou l'alliage de métaux dégradable constitue un alliage biocorrodable, comprenant du magnésium, du fer, du zinc et/ou du tungstène.

6. Stent selon la revendication 5, **caractérisé en ce que** l'alliage est un alliage de magnésium.

7. Stent selon la revendication 5 ou 6, **caractérisé en ce que** l'alliage de magnésium comprend de l'yttrium.

8. Stent selon l'une des revendications précédentes, **caractérisé en ce que** la couche de séparation b) comprend ou est constituée de carbure de silicium (SiC), de parylènes (poly-para-xylylène), glycocalyx, le polysulphone, le caoutchouc de silicone, le polyuréthane, l'hydroxylapitite et/ou un diamant comme le carbone.

9. Stent selon l'une des revendications précédentes, **caractérisé en ce que** la couche de séparation b) présente une épaisseur de 100 nm à 5 µm.

10. Stent selon l'une des revendications précédentes, **caractérisé en ce que** la ou les substances actives sont appropriées dans la prévention et/ou le traitement de la resténose intra-stent et/ou de l'inflammation des tissus et/ou sont sélectionnés à partir du groupe comprenant :
les lipidorégulateurs, les immunosuppresseurs, les vasodilatateurs, les inhibiteurs des canaux calciques, les inhibiteurs de la calcineurine, les antiphlogistiques, les anti-inflammatoires, les antiallergiques, les oligonucléotides, les estrogènes, les générateurs d'endothélium, les stéroïdes, les protéines, les peptides, les bloqueurs de prolifération, les analgésiques, les antirhumatismaux et les cytostatiques.

11. Procédé pour la fabrication d'un stent selon l'une des revendications précédentes, comprenant ou constitué des étapes suivantes :
a) mise à disposition d'un corps de base de stent métallique dégradable et d'une première préparation comprenant une ou plusieurs substances,
b) revêtement de la surface du corps de base de stent avec la première préparation, de manière à former une couche de séparation, dans lequel au moins des parties de la surface de la face luminale ne sont pas recouvertes,
c) mise à disposition d'une deuxième préparation comprenant une ou plusieurs substances actives et le cas échéant un ou plusieurs copolymères, et
d) revêtement de la surface ou de parties de la surface de la couche de séparation issue de l'étape b) sur la face abluminale du corps de base de stent avec la deuxième préparation c), de manière à former une couche contenant des substances actives.

12. Procédé selon la revendication 11, **caractérisé en ce que** dans l'étape b), la surface du corps de base de stent est recouverte avec la première préparation sur la face abluminale, de manière à former la couche de séparation b) sur la face abluminale du corps de base de stent et à ce que la face luminale ne soit pas recouverte avec la couche de séparation b).

13. Procédé selon la revendication 11 ou 12, **caractérisé en ce que** le procédé comprend en outre les étapes suivantes :
e) mise à disposition d'une troisième préparation comprenant un ou plusieurs polymères, et
f) revêtement de la surface du stent réalisé selon la revendication 11 ou 12 avec la préparation, de manière à former une troisième couche polymère recouvrant la surface ou des parties de la surface de la couche contenant des substances actives d) et/ou de la couche de séparation b) et/ou du couche de séparation sans revêtement.
